# EUROPEAN PATENT SPECIFICATION

(11) **EP 2 193 769 B1**
(45) Date of publication and mention of the grant of the patent: **28.08.2013**
(21) Application number: 08765005.7
(22) Date of filing: 03.06.2008
(51) Int. Cl.: A61F 13/02, A61K 9/70, A61K 47/32, A61K 47/34, A61L 15/58, B32B 25/08, C09J 7/02, A61K 8/02, A61Q 19/08

(54) **PATCH MATERIAL**
PFLASTERMATERIAL
MATÉRIAU DE TIMBRE

(30) Priority: 28.09.2007 JP 2007256570
(43) Date of publication of application: 09.06.2010
(73) Proprietor: Nichiban Co. Ltd., Tokyo 112-8663 (JP)
(72) Inventor: FUKANO, Kenji, Tokyo 112-8663 (JP); FUJISAWA, Hiromichi, Tokyo 112-8663 (JP); WATANABE, Shuichi, Tokyo 112-8663 (JP)
(74) Representative: Jones, Helen M.M.
(86) International application number: PCT/JP2008/060192
(87) International publication number: WO 2009/041122

(56) References cited:
- EP-A1- 1 177 786
- EP-A2- 1 025 864
- WO-A2-2007/109593
- JP-A- 7 132 139
- JP-A- 7 255 772
- JP-A- 2000 217 858
- JP-A- 2007 068 723

## Description

### TECHNICAL FIELD

The present invention relates to a patch having a layer structure that a pressure sensitive adhesive layer is provided on one surface of a base layer. In the patch according to the present invention, both layers of the base layer and pressure sensitive adhesive layer making up the patch closely adheres to an adherend along the surface having fine irregularities of the adherend, so that a difference in surface condition between a stuck portion and an unstuck portion is small, and so the stuck portion is inconspicuous.

When the patch according to the present invention is used as a patch for human skin, the patch closely adheres to the skin along the skin surface having fine irregularities such as skin furrows in a state as if a fine texture structure of the skin surface would be transferred to a back surface (surface of the base layer on the side reversed to the pressure sensitive adhesive layer) of the patch through the patch, and so the stuck portion is hard to be conspicuous.

Since the patch according to the present invention is thin, rich in stretchability and easily follows the movement of the skin in a stuck state, a feeling of physical disorder during sticking is greatly relieved. Since the patch according to the present invention has moderate adhesive strength, the patch is excellent in balance between adhesion to the skin surface and releasability after use.

### BACKGROUND ART

A patch is stuck for use on various adherends in forms suitable for respective uses as various patches for industries, utensils, medical cares, household and the like. Patches (skin patches) for human skin that the surface of the human skin is intended as an adherend include medical patches typified by dressing materials for protecting the skin with a damaged site, operation scar or the like; and household patches such as first-aid adhesive bandages, waterproofing patches and protecting patches for protecting the skin. These household patches are also used in medical scenes because of protecting the skin with a damaged site or a hole pierced by a syringe needle or the like.

A patch generally has a layer structure composed of a base layer formed of a plastic film, woven fabric, nonwoven fabric, knit fabric, paper or the like, and a pressure sensitive adhesive layer provided on at least one surface of the base layer. Since the patch is a foreign matter to an adherend, the patch is required to be inconspicuous in a stuck state in many uses. In particular, a skin patch often stuck on the surface of the human skin exposed is strongly required to make a stuck portion inconspicuous.

In order to make the stuck portion hard to be inconspicuous, the patch is preferably caused to closely adhere to the skin along the skin surface having fine irregularities such as skin furrows. If the patch is stuck on the skin without closely adhering to the finely irregular surface in a state as if it would rise above the skin, a difference in appearance between the patch and the skin surface around it is marked, and so the stuck portion is conspicuous. In order to make the stuck portion hard to be inconspicuous, it is preferable that the patch closely adheres to the skin along the irregular surface with skin furrows or the like of a fine texture structure, and the texture structure appears on a surface (back surface of the base layer) of the patch in a state as if it would be transferred to the surface.

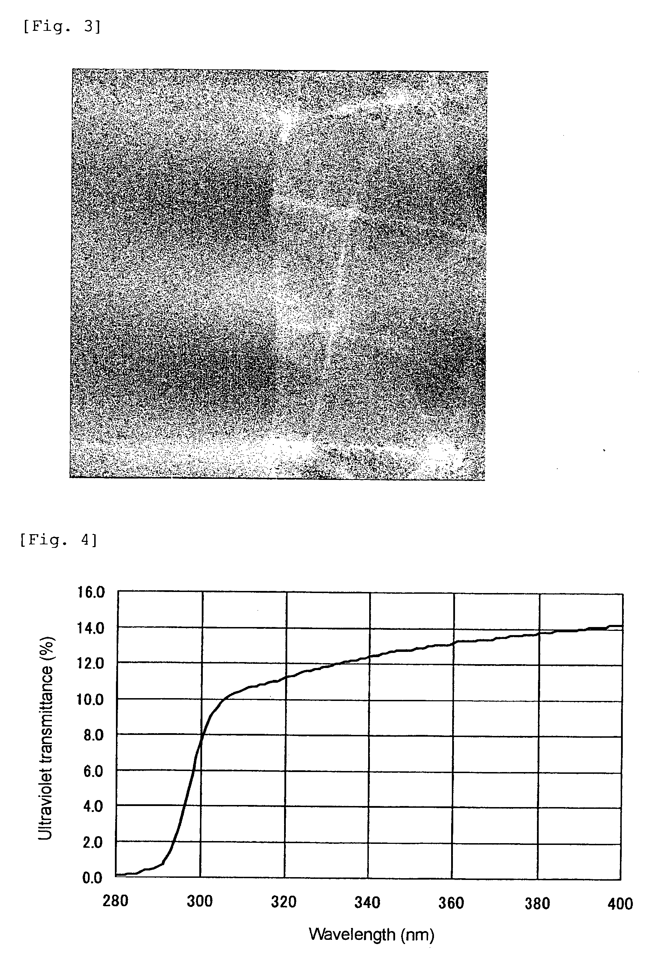

FIG. 3 illustrates an image (photograph) through a confocal microscope obtained at the time a patch (Comparative Example 4 of the present description), in which the thickness of a base layer exceeds 15 µm, and the total thickness of the base layer and a pressure sensitive adhesive layer exceeds 20 µm, has been stuck on the surface of the human skin. More specifically, FIG. 3 illustrated an image obtained by making a model of the stuck state with an impression material used in making a tooth form upon treatment in dental surgery in a state that the patch has been stuck on the skin surface and observing the model through the confocal microscope. A left half of FIG. 3 shows the surface (back surface of the base layer) of the patch, and a right half thereof shows the fine texture structure of the surface of the human skin. As apparent from FIG. 3, the patch does not closely adhere to the finely irregular surface of the skin in a stuck state and becomes a state as if it would rise above the skin, and the fine texture structure on the skin surface is not in the state transferred to the back surface of the patch, so that the stuck portion is liable to be conspicuous.

In the skin patch, it is also an important subject not to give a feeling of physical disorder during sticking. If the patch is hard to easily follow the movement of the skin, a feeling of physical disorder attributed to the resistance of the patch is caused. In order not to give the feeling of physical disorder during sticking, the patch is required to have stretchability so as to easily follow the movement of the skin surface having fine irregularities.

The total thickness of a patch is thinned, and a base having stretchability is used, whereby the patch is expected to have effects of making a stuck portion inconspicuous when the patch is stuck on the skin surface, and relieving the feeling of physical disorder during sticking. However, according to the results of researches by the present inventors, it has been proved that it is difficult to obtain desired results simply by thinning the thickness of the patch or using a stretchable base.

The patch is required to have moderate adhesive strength. If the adhesive strength of the patch is too weak, the patch is simply peeled from the skin surface or cannot closely adhere to the skin along the skin surface having fine irregularities such as skin furrows. If the adhesive strength of the patch is too strong, a rash may be caused, or peeling of the patch after use becomes difficult. When each thickness of the base layer, pressure sensitive adhesive layer and patch is thinned, it is liable to encounter a difficulty in achieving the moderate adhesive strength.

If the base layer making up the patch is strong in stiffness and insufficient in stretchability, the patch cannot easily follow the movement of the skin surface. When the thicknesses of the base layer and pressure sensitive adhesive layer are thinned, the stretchability of the patch can be enhanced. However, the patch is liable to lower adhesion to the skin surface.

Therefore, the skin patch is required to balance various characteristics such as inconspicuousness of a stuck portion, relief of a feeling of physical disorder during sticking and moderate adhesive strength with one another at a high level by synthetically investigating the stretchability of the patch, the stretchability of the base layer, the adhesive strength of the pressure sensitive adhesive layer, the thickness of each layer, the material of the base layer, and the like.

In the prior art on patches, such viewpoints as described above have been lacked, or research and development from such viewpoints as described above have been insufficient. Patches stressing the inconspicuousness of the stuck portion are marketed. However, the lower limit value of the thickness of the base layer is from about 20 µm to about 30 µm in most of them. When a stuck portion of such a patch is observed, it exhibits appearance clearly different from an unstuck portion, and so the stuck portion is conspicuous. In addition, many of them give a feeling of physical disorder because they do not easily follow the movement of the skin when stuck on the skin surface.

Japanese Patent Application Laid-Open No. 6-336425 (Patent Art. 1) has proposed an analgesic-hemostatic patch, in which a pressure sensitive adhesive layer containing an analgesic and a hemostat and having a thickness of 5 to 350 µm is formed on one surface of a support having visible ray permeability and a thickness of 5 to 150 µm. Respective Examples of Patent Art. 1 show an analgesic-hemostatic patch, in which an acrylic pressure sensitive adhesive layer having a thickness of 20 µm is provided on an ethylene-vinyl acetate copolymer (EVA) film having a thickness of 60 µm; and an analgesic-hemostatic patch, in which a rubbery pressure sensitive adhesive layer having a thickness of 200 µm or 300 µm is provided on an EVA film having a thickness of 60 µm. Such an analgesic-hemostatic patch having a great thickness is conspicuous in a stuck portion in a stuck state and gives a strong feeling of physical disorder.

Japanese Patent Application Laid-Open No. 9-301853 (Patent Art. 2) has proposed a patch preparation, in which an inelastic layer having a thickness of 10 to 100 µm and a pressure sensitive adhesive layer having a 1 to 15 µm are successively formed on one surface of a support having a thickness of 0.5 to 20 µm. In the patch preparation described in Patent Art. 2, a silicone pressure sensitive adhesive layer or acrylic pressure sensitive adhesive layer having a great thickness is arranged as the inelastic layer, so that a stuck portion is conspicuous, and a feeling of physical disorder during sticking is strong.

Japanese Patent Application Laid-Open No. 7-255772 (Patent Art. 3) discloses a base for skin patch composed of an elastomer resin film having a thickness of 5 to 20 µm. However, Patent Art. 3 does not specifically disclose the thickness and adhesive strength of a pressure sensitive adhesive layer formed on the base. Patent Art. 3 does not propose inconspicuousness of a stuck portion, relief of a feeling of physical disorder when stuck on the skin, adhesive strength and the like as the whole patch including the pressure sensitive adhesive layer on synthetic investigation.

International Publication No. WO91/16044 (Pamphlet) (Patent Art. 4) has proposed a patch composed of a film layer formed of film having a thickness of 0.5 to 4.9 µm, each strength in 2 directions substantially perpendicular to each other of 8 to 85 g/mm, each elongation in 2 directions substantially perpendicular to each other of 30 to 150% and a ratio between the elongations in the 2 directions of 1.0 to 5.0, and a pressure sensitive adhesive layer formed of a pressure sensitive adhesive laminated on one surface of the film layer, having a thickness of 2 to 60 µm and containing a percutaneously absorbable drug.

Films specifically disclosed in Patent Art. 4 are resin films such as a polyethylene terephthalate (PET) film, which are small in elongation at break (elongations of films used in Examples: 120% or less) and high in glass transition temperature (glass transition temperature of PET: 76 to 77°C). The resin films such as the PET film is rigid and insufficient in stretchability. Therefore, the patch described in Patent Art. 4 is hard to closely adhere to the skin along the skin surface having fine irregularities such as skin furrows, liable to be conspicuous in a stuck portion and gives a strong feeling of physical disorder during sticking. If the thickness of the PET film is 5 µm or more, such a patch is liable to induce a rash on the skin (Patent Art. 4, page 13, right lower column), so that there is a great restriction in the designing of the patch.

Japanese Patent Application Laid-Open No. 8-40910 (Patent Art. 5) discloses a patch, in which an acrylic pressure sensitive adhesive layer having a thickness of 10 µm or more, preferably from 20 µm or more to 200 µm or less is provided on a PET film having a thickness of 0.5 to 6 µm. However, this patch is liable to be conspicuous in a stuck portion and gives a strong feeling of physical disorder during sticking as described above even if the thickness of the base layer is thin, since the base layer is the PET film.

Japanese Patent Application Laid-Open No. 2005-218496 (Patent Art. 6) has proposed a skin patch, in which a pressure sensitive adhesive layer is formed on one surface of a base film formed of an ether type urethane resin and having a thickness of 10 to 50 µm, preferably 25 to 35 µm. Examples of Patent Art. 6 disclose skin patches, in which an acrylic pressure sensitive adhesive layer having a thickness of 30 µm was formed on one surface of an ether type polyurethane resin film having a thickness of 30 µm. However, the skin patches specifically disclosed in Patent Art. 6 are great in thickness and insufficient in stretchability, so that a stuck portion is liable to be conspicuous and a feeling of physical disorder during sticking is not sufficiently relieved.

Japanese Patent No. 3868542 (Patent Art. 7) discloses a hiding seal, in which the glass transition temperature of a base film is 25 to 35°C, and the water vapor transmission rate of a laminate of the base film and a pressure sensitive adhesive layer is 1,000 g/m²·24 hr or more. However, the base film specifically disclosed in Patent Art. 7 is hard to closely adhere to the skin along the skin surface having fine irregularities such as skin furrows because of being rigid at ordinary temperature.

The conventional patches are hard to closely adhere to the skin along the skin surface having fine and shallow skin furrows like in the human forearm or the face and cannot greatly reduce ease of conspicuousness at a stuck portion. In addition, it is difficult to satisfactorily achieve relief of a feeling of physical disorder during sticking.

Under the circumstances, the thickness, material and properties of the base layer making up the patch; the thickness and adhesive strength of the pressure sensitive adhesive layer; the thickness and properties of the whole patch; and the like have heretofore been not synthetically investigated from the viewpoints of inconspicuousness of a stuck portion, relief of a feeling of physical disorder during sticking, and the like. Therefore, there is a demand for research and development of a new patch from such viewpoints.

Patent Art. 1: Japanese Patent Application Laid-Open No. 6-336425
Patent Art. 2: Japanese Patent Application Laid-Open No. 9-301853
Patent Art. 3: Japanese Patent Application Laid-Open No. 7-255772
Patent Art. 4: International Publication WONo. 91/16044 (Pamphlet)
Patent Art. 5: Japanese Patent Application Laid-Open No. 8-40910
Patent Art. 6: Japanese Patent Application Laid-Open No. 2005-218496
Patent Art. 7: Japanese Patent No. 3868543

### DISCLOSURE OF THE INVENTION

### PROBLEMS SOUGHT FOR SOLUTION BY THE INVENTION

It is an object of the present invention to provide a patch, which closely adheres to an adherend along the finely irregular surface of the adherend, is hard to be conspicuous in a stuck portion and is easily stretchable according to the movement of the adherend.

In particular, an object of the present invention is to provide a patch, in which when the patch is used as a patch for human skin, the patch closely adheres to the skin along the skin surface having fine irregularities such as skin furrows and is inconspicuous in a stuck portion, and moreover the patch easily follows the fine movement of the skin while retaining the stuck state and can greatly relieve a feeling of physical disorder during sticking.

The present inventors have considered that when a patch closely adheres to the skin along the skin surface having fine irregularities such as skin furrows, and a fine texture structure of the skin surface is in a state transferred to a back surface of the patch through the patch, a stuck portion is hard to be conspicuous. In addition, the present inventors have considered that when a base layer is formed by a film thin and rich in stretchability, and the thickness of a pressure sensitive adhesive layer is also thinned, the resulting patch easily follows the movement of the skin in a stuck state, so that a feeling of physical disorder during sticking is greatly relieved.

However, it has been proved that a patch obtained by forming a base layer with a thin polyethylene terephthalate film (PET film) having a thickness of 5 µm or less and also thinning the thickness of a pressure sensitive adhesive layer to 5 µm does not sufficiently closely adhere to the skin along the skin surface having fine irregularities such as skin furrows, and a stuck state is conspicuous. It has also been found that this patch lacks following ability to the movement of the skin, so that a feeling of physical disorder during sticking is great.

The present inventors have found that it is difficult to reconcile inconspicuousness and relief of a feeling of physical disorder when stretchability of a patch in a plane direction is insufficient, or the relationship between stretchability and the thickness of the patch or the thickness of a base layer is not within a specific range even when the base layer is formed with a film having flexibility and stretchability, and each thickness of the base layer and pressure sensitive adhesive layer is thin, to say nothing of the case where both layers are thick.

In order for a patch to close adhere to an adherend along the surface having fine irregularities and to easily follow the movement of the adherend, the patch is required to be easily stretched in the plane direction. In order for a patch in a flat form to closely adhere to an adherend along the surface having fine irregularities like the skin surface, the patch is required to be easily stretched in the plane direction so as to flexibly elongate along the finely irregular surface.

In general, in order to stick a wide-area patch on the surface of an adherend having fine irregularities, there is conducted an operation that the patch is first fixed to convex portions of the adherend's surface, and the patch is put in along the surfaces of concave portions of the adherend from the convex portions as points of origin. In order for the patch to closely adhere to the concave surfaces of the adherend, the patch is required to have sufficient ease of elongation in the plane direction. If the patch is hard to elongate in the plane direction, the patch comes to be stuck on the adherend in a state risen above the concave portions, so that a stuck portion is liable to be conspicuous. In addition, if the patch is hard to elongate in the plane direction, the form of fine irregularities such as skin furrows is deformed, whereby a difference in appearance from the skin in the vicinity of a stuck portion may be made in some cases to make it liable to be conspicuous.

The ease of elongation of the patch in the plane direction not only gives good following ability to the irregular surface, but also relates to a feeling of physical disorder by the patch upon sticking on the skin. On the surface of the skin, expansion and contraction frequently occur. If the resistance to stretching of the patch is great, a feeling of resistance is created upon expansion and contraction of the skin. This feeling of resistance comes to be felt as a feeling of physical disorder by the patch during sticking. When the patch is easy to elongate in the plane direction, the patch easily follows the movement of the skin surface, so that the feeling of physical disorder comes to be relieved.

The skin not only moves so as to elongate, but also contracts by the elongate amount so as to return to an original form. If the patch only elongates in the plane direction and does not return to the original form, the resistance of the patch as it elongated is felt upon contraction of the skin, so that it comes to feel a feeling of physical disorder. In order to lessen the feeling of physical disorder in the state stuck to the skin as much as possible, the patch is required to have good stretchability.

In view of the problems involved in the prior art, the present inventors have considered that not only attention is paid to the thicknesses of a patch and respective layers making up the patch, stretchability of a base layer, and the like, but also a synthetic investigation as to (1) the thickness, material and properties of the base layer, (2) the thickness and adhesive strength of a pressure sensitive adhesive layer, (3) the total thickness of the base layer and the pressure sensitive adhesive layer and properties of a pressure sensitive adhesive, and (4) interrelation among these is required for achieving the above objects.

The present inventors have found that it is effective to use an extremely thin elastomer film as a base layer and thin the thickness of the base layer and the total thickness of the base layer and a pressure sensitive adhesive layer for achieving the above objects. When the thickness of a patch is sufficiently thin compared with the size of irregularities on the surface of an adherend, the patch can be caused to closely adhere to the adherend along the irregular surface thereof. For example, the thickness of a patch is as thin as a fifth of the width of a concave portion in the surface of the adherend, such a patch can be caused to closely adhere to the adherend along the surface having fine irregularities.

In order to cause a patch to closely adhere to the surface of an adherend, a pressure sensitive adhesive layer is required to have moderate adhesiveness to the adherend. On the other hand, if the adhesive strength of the pressure sensitive adhesive layer is excessively great, difficulty is encountered on peeling after use.

In addition, the present inventors have conceived that in order to obtain a patch, which closely adheres to an adherend along the surface having a fine texture structure of the adherend like the human skin surface and easily follows the movement of the adherend, it is important that a 10% tensile load in each of 2 directions (machine and transverse directions) perpendicular to each other of the patch is sufficiently small, and the product of the 10% tensile load of the patch and the thickness of the patch and/or the product of the 10% tensile load of the patch and the thickness of the base layer falls within a specific range in addition to thinning of the thicknesses of the patch and the respective layers making up the patch.

The present invention has been led to completion on the basis of these findings.

### MEANS FOR THE SOLUTION OF THE PROBLEMS

According to the present invention, there is provided a patch having a layer structure that a pressure sensitive adhesive layer is provided on one surface of a base layer, wherein
(a) the base layer is a polyurethane elastomer film having a thickness within a range of from 1 to 8 µm,
(b) the thickness of the pressure sensitive adhesive layer is within a range of from 1 to 9 µm,
(c) the total thickness of the base layer and the pressure sensitive adhesive layer is within a range of from 2 to 15 µm,
(d) the 10% tensile load of the patch in each of machine and transverse directions is within a range of from 0.03 to 1.1 N/10 mm as measured in accordance with Japanese Industrial Standard JIS Z 0237,
(e) assuming that the 10% tensile load value of the patch in the machine direction is X (N/10 mm), the thickness value of the patch is Y (µm), and the thickness value of the base layer is Z (µm), the patch satisfies the relationship that the product XY value of the 10% tensile load value X and the thickness value Y of the patch falls within a range of from 0.10 to 12, or the product XZ value of the 10% tensile load value X and the thickness value Z of the base layer falls within a range of from 0.05 to 6.8, and
(f) the pressure sensitive adhesive layer exhibits adhesive strength of 0.15 to 2 N/10 mm or more in a 90-degree peeling test to a Bakelite plate prescribed in Japanese Industrial Standard JIS Z 0237, and
(g) the pressure sensitive adhesive layer is any one selected from the group consisting of acrylic pressure sensitive adhesive layer, natural rubber-based pressure sensitive adhesive layer, synthetic rubber-based pressure sensitive adhesive layer, silicone-based pressure sensitive adhesive layer, vinyl ester-based pressure sensitive adhesive layer, vinyl ether-based pressure sensitive adhesive layer and urethane-based pressure sensitive adhesive layer.

### EFFECTS OF THE INVENTION

According to the present invention, there can be provided a patch that a feeling of physical disorder in a stuck state is extremely small both visually and sensuously. In the patch according to the present invention, both layers of the base layer and pressure sensitive adhesive layer making up the patch closely adheres to an adherend along the surface having fine irregularities of the adherend, so that a difference in surface condition between a stuck portion and an unstuck portion is small, and so the stuck portion is hard to be conspicuous. The patch according to the present invention closely adheres to the skin along the skin surface having fine irregularities such as skin furrows to create a state that a fine texture structure of the skin surface has been transferred to a back surface of the patch through the patch, and so the stuck portion is hard to be conspicuous.

Since the patch according to the present invention is thin, rich in stretchability and easily follows the expanding and contracting movement of the skin in a stuck state, a feeling of physical disorder during sticking is greatly relieved. Since the patch according to the present invention has moderate adhesive strength, the patch is excellent in balance between adhesion to the surface of the adherend such as the skin and releasability after use.

The patch according to the present invention can be utilized as various patches for medical cares, makeup, industries, utensils, household and the like making the best use of these features.

### BRIEF DESCRIPTION OF THE DRAWINGS

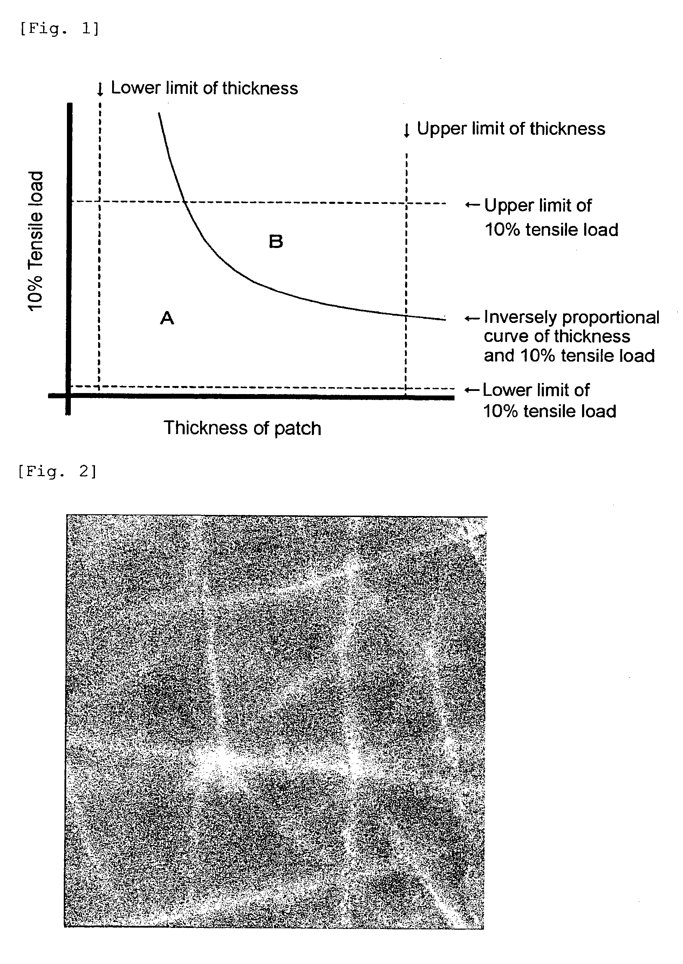

FIG. 1 is a conceptual diagram illustrating the relationship between the thickness of a patch and a 10% tensile load.

FIG. 2 illustrates an image through a confocal microscope obtained by sticking a patch (in which the thickness of a base layer is 1 µm, and the thickness of a pressure sensitive adhesive layer is 2 µm) prepared in Example 4 on the surface of the human skin, and using a model obtained by transferring the stuck state to an impression material for dental surgery. A left half of FIG. 2 shows a back surface of the patch, and a right half thereof shows the fine structure of the skin surface.

FIG. 3 illustrates an image through a confocal microscope obtained by sticking a patch (in which the thickness of a base layer is 18 µm, and the thickness of a pressure sensitive adhesive layer is 7 µm) prepared in Comparative Example 4 on the surface of the human skin, and using a model obtained by transferring the stuck state to an impression material for dental surgery. A left half of FIG. 3 shows a back surface of the patch, and a right half thereof shows the fine structure of the skin surface.

FIG. 4 is a graph illustrating an ultraviolet transmittance within a wavelength range of from 280 to 400 nm of a patch obtained in Example 1.

### BEST MODE FOR CARRYING OUT THE INVENTION

### 1. Layer structure of patch

The patch according to the present invention has a layer structure that a pressure sensitive adhesive layer is provided on one surface of a base layer. Additional layers such as a carrier layer and a separator layer may be provided on the patch according to the present invention in addition to these layers. Such additional layers are peeled upon sticking. In the present invention, the properties (for example, 10% tensile load, ultraviolet transmittance and water vapor transmission rate) of the patch mean properties on a laminate composed of 2 layers of the base layer and pressure sensitive adhesive layer.

A separator layer is arranged in many of patches for protecting the pressure sensitive adhesive layers thereof. It is preferable to adopt a process of forming a pressure sensitive adhesive layer on a separator layer not only from the viewpoint of the protection of the pressure sensitive adhesive layer but also for forming a thin and uniform pressure sensitive adhesive layer.

On the other hand, a base layer is often used in the form of a single layer. However, when the thickness thereof is extremely thin like the base layer in the patch according to the present invention, it is preferable to adopt a process of forming a base layer on a carrier layer.

A laminate obtained by forming a pressure sensitive adhesive layer on one surface of a separator layer and a laminate obtained by forming a base layer on one surface of a carrier layer are laminated on each other, whereby a patch having a laminated structure of "carrier layer/base layer/pressure sensitive adhesive layer/separator layer", in which the carrier layer is arranged on the surface of the base layer on the side reversed to the pressure sensitive adhesive layer, and the separator layer is arranged on the surface of the pressure sensitive adhesive layer on the side reversed to the base layer, can be produced.

A patch is easily produced by adopting the above-described laminated structure, and moreover the handling property of the resultant patch is improved. A patch, which is composed of a base layer and a pressure sensitive adhesive layer each having a thin thickness, in the form that neither a carrier layer nor a separator layer is arranged is weak in stiffness and liable to wrinkle, and so such a patch is difficult to be handled. In the patch having the laminated structure, the separator layer is peeled upon sticking to expose the pressure sensitive adhesive layer. Even when the separator layer is peeled, the stiffness of the patch is strong because the carrier layer is present, and so the surface of the pressure sensitive adhesive layer can be brought into contact with a desired sticking site. After sticking, the carrier layer on the base layer is also peeled.

### 2. Production process of Patch

An elastomer can be formed into a film by any forming process such as a solution casting process, extrusion process, calendering process or blown-film extrusion process. Since the base layer of the present invention is an elastomer film having an extremely thin thickness within a rage of from 1 to 10 µm, the solution casting process (solution coating process) or extrusion laminating process is preferably adopted for stable and continuous production while inhibiting occurrence of break, and the solution casting process is more preferably adopted.

In the solution casting process, a process, in which a solution of an elastomer in an organic solvent is cast on a support, and the organic solvent is dried, is preferably adopted. When a carrier layer is used as the support, and the elastomer solution is cast thereon while running the carrier layer in one direction and dried, an elastomer film can be continuously formed. According to the solution casting process, the thickness of the elastomer film can be accurately controlled, and an elastomer film small in directional anisotropy of physical properties in the film can be formed.

As the extrusion process, is preferred an extrusion laminating process (extrusion lamination). Examples of the extrusion laminating process include a process, in which a polymer material forming a support (carrier layer) and an elastomer are co-extruded from a T-die into films, and the films are laminated; and a process, in which an elastomer is melt-extruded on a support (carrier layer) from a T-die while running the support in one direction to laminate them.

The running direction of the carrier layer is called a machine direction (MD) and a direction perpendicular to this direction on a plane is called a transverse direction (TD). The machine direction of the elastomer film is determined on the basis of the running direction of the carrier layer or the extruding direction (MD) of an extruder. With respect to a patch using an elastomer film as a base layer, the machine direction and transverse direction of the patch are determined according to a distinction between the machine direction and the transverse direction of the elastomer film.

A process, in which a solution of a pressure sensitive adhesive is cast on a separator layer and dried, is preferably adopted for forming a pressure sensitive adhesive layer. As a continuous forming process of the pressure sensitive adhesive layer, is preferred a process, in which a solution of a pressure sensitive adhesive is cast on a separator layer while running the separator layer, and dried. A process, in which a pressure sensitive adhesive is melted and applied on to a separator layer, may also be adopted.

A laminate with a pressure sensitive adhesive layer formed on one surface of a separator layer and a laminate with a base layer formed on one surface of a carrier layer are separately formed, and these laminates are then laminated in such a manner that the pressure sensitive adhesive layer comes into close contact with the surface of base layer, whereby a patch having a laminated structure of "carrier layer/base layer/pressure sensitive adhesive layer/separator layer" can be produced.

In order to improve the touch, slip property, appearance and the like of the patch when the patch is stuck on the surface of the human skin, fine irregularities may be preferably formed on the back surface (surface of the base layer on the side reversed to the pressure sensitive adhesive layer) of the base layer making up the patch in some cases. In such a case, the fine irregularities are formed on the surface of the carrier layer by embossing, and the base layer is then formed on the finely irregular surface of the carrier layer, whereby the fine irregularities can be transferred to the surface (back surface) of the base layer formed of the elastomer film.

### 3. Base layer

In the present invention, an elastomer film having a thickness within a range of from 1 to 8 µm is used as the base layer.

As an elastomer used for forming the elastomer film, can be used an elastomer having excellent stretchability and satisfying such requirements that (1) a thin film within a range of from 1 to 8 µm can be formed, (2) when the film is used as a base layer of a patch, a patch, whose 10% tensile load in each of machine and transverse directions is within a range of from 0.03 to 1.1 N/cm, can be produced, and (3) when the film is used as a base layer of a patch, the XY value and/or XZ value of the patch satisfies the specific range. The 10% tensile load of the patch is substantially equal to the 10% tensile load of the elastomer film forming the base layer because the pressure sensitive adhesive layer is thin.

As examples of the elastomer, may be mentioned polyurethane elastomers, 1,2-polybutadiene type thermoplastic elastomers, polystyrene type thermoplastic elastomers, polyolefin type thermoplastic elastomers and mixtures of 2 or more elastomer thereof.

Among these elastomers, a polyurethane elastomer is preferred from the viewpoints of excellent thin-film-forming property, excellent stretchability of the resulting film and easy control of the 10% tensile load value, XY value and XZ value of the resulting film within desired respective ranges.

The polyurethane elastomer is an elastomer having a urethane group in its molecule and is formed by a polyaddition reaction of a polyol component and an isocyanate component. As the polyol component, is used a polyol compound having two or more OH groups, and a long-chain diol is used in many cases. In addition, a short-chain diol acting as a chain-lengthening agent may be used in combination as a polyol component in some cases. Besides, a monomolecular polyol such as trimethylolpropane, glycerol or sorbitol may be used as a crosslinking agent in some cases. As the isocyanate component, is used a polyisocyanate compound having two or more NCO groups, and a diisocyanate is used in many cases. A production technique of the polyurethane elastomer is a technique well-known in the art.

The polyurethane elastomer includes thermoplastic polyurethane elastomers, and thermosetting polyurethane elastomers (amine-cured polyurethane elastomer and OH-cured polyurethane elastomer), and no particular limitation is imposed thereon. The thermoplastic polyurethane elastomers include a complete thermoplastic polyurethane elastomer, in which the NCO group is scarcely present, and an incomplete thermoplastic polyurethane elastomer, in which a considerable amount of the NCO group remains, and partial intermolecular crosslinking occurs upon forming or molding. In the present invention, any elastomer may be used. The thermosetting polyurethane elastomers include a one-component type and a two-component type, and any type may be used. However, one-component type is preferred from the viewpoint of easy film formation.

In the polyurethane elastomer, various classification methods are present. However, the nature of the polyurethane elastomer is greatly affected by the kind of the polyol component making up a soft segment, so that the elastomer is conveniently classified according to the kind of the polyol component.

More specifically, the polyurethane elastomer includes (1) a caprolactone type polyurethane elastomer synthesized by a polyaddition reaction of a polylactone ester polyol obtained by ring-opening polymerization of caprolactone, and a diisocyanate, (2) an adipic acid ester type polyurethane elastomer synthesized by a polyaddition reaction of an adipic acid ester polyol of adipic acid and a glycol, and a diisocyanate, and (3) a polyether type polyurethane elastomer synthesized by a polyaddition reaction of a polyether polyol composed of polytetramethylene glycol obtained by ring-opening polymerization of tetrahydrofuran, or polyalkylene glycol such as polypropylene glycol, and a diisocyanate. In these polyurethane elastomers, a polyol having 3 or more OH groups and/or a polyisocyanate having 3 or more NCO groups may also be used.

The polyurethane elastomer can be classified into a polyether type and a polyester type according to the structure of a main chain. Examples of the polyether type polyurethane elastomer include those described above. Examples of the polyester type polyurethane elastomer include the above-described caprolactone type polyurethane elastomer and adipic acid ester type polyurethane elastomer. However, the polyurethane elastomer used in the present invention is not limited by the structure of the main chain, and any other type polyurethane elastomer may also be used.

As examples of commercially available products of the polyurethane elastomer suitable for use in the present invention, may be mentioned LUCKSKIN US2268 (polyether type); and LUCKSKIN U-1223, U-1285 and U-2860 (polyester type) available from SEIKOH CHEMICALS CO., LTD. However, it is not limited thereto. These polyurethane elastomers may be used singly or in combination of 2 or more elastomers thereof.

No particular limitation is imposed on the glass transition temperature (Tg) of the elastomer. For example, the glass transition temperature of a high-modulus polyurethane elastomer may be as high as 50°C. The glass transition temperature of the elastomer is preferably within a range of from -70°C to 20°C from the viewpoints of stretchability, flexibility and 10% tensile load of the resulting elastomer film. The upper limit value of the glass transition temperature is preferably 20°C, more preferably 10°C, particularly preferably 0°C. The lower limit value of the glass transition temperature is -60°C or -55°C in many cases. The glass transition temperature is a value measured by means of a differential scanning calorimeter in accordance with a method known *per se* in the art.

The base layer of the present invention is an elastomer film having a thickness of 1 to 8 µm. The elastomer film is preferably small in directional difference (anisotropy) of physical properties from the viewpoint of a little feeling of physical disorder (a feeling of resistance of the patch felt at the time the skin has extended and contracted) during sticking. A solution casting process is preferably adopted as the film-forming process because a thin elastomer film, in which physical properties such as 10% tensile load are substantially the same in both machine direction and transverse direction, can be obtained.

The thickness of the elastomer film forming the base layer is within a range of from 1 to 8 µm. The thickness of the elastomer film can be thinned up to a range of from 1 to 5 µm from the viewpoints of inconspicuousness and relief of a feeling of physical disorder of the resulting patch during sticking.

If the thickness of the elastomer film is less than 1 µm, its strength as the base layer becomes insufficient in addition to difficulty in forming such a film, and so the base layer may be cut in some cases upon sticking of the resulting patch on an adherend or peeling of the patch from the adherend. If the thickness of the elastomer film is too great, the resulting patch is hard to closely adhere to the skin along the skin surface having fine irregularities such as skin furrows even when the thickness of the whole patch is thinned, a stuck state is liable to be conspicuous, and there is a tendency for a feeling of physical disorder to become great.

In order to control the 10% tensile load of a patch in each of machine and transverse directions within a range of from 0.03 to 1.1 N/10 mm, an elastomer film, whose 10% tensile load in each of machine and transverse directions falls within a range of from 0.03 to 1.1 N/10 mm, is used as the base layer.

The 10% tensile load of the elastomer film in each of machine and transverse directions as measured in accordance with Japanese Industrial Standard JIS Z 0237 is within a range of from 0.03 to 1.1 N/10 mm, more preferably from 0.05 to 1.0 N/10 mm, particularly preferably from 0.06 to 0.95 N/10 mm.

In case of an elastomer film small in directional anisotropy of physical properties like the film obtained according to the solution casting process, the 10% tensile load value thereof in a machine direction is substantially the same as that in a transverse direction. Even by any other film-forming process, the 10% tensile load values of the resulting elastomer film in the machine and transverse directions are not greatly different.

If the 10% tensile load of the elastomer film is too small, the thickness thereof is generally too small, so that film-forming property and handling property are lowered. If the 10% tensile load of the elastomer film is too great, the rigidity thereof becomes strong, and the stretchability and flexibility thereof are hence insufficient, so that a patch obtained by using such an elastomer film as the base layer is hard to closely adhere to the skin along the skin surface having fine irregularities such as skin furrows, and a stuck state is liable to be conspicuous. In addition, the patch obtained by using the elastomer film too great in the 10% tensile load as the base layer gives a strong feeling of physical disorder upon sticking on the skin surface.

The 10% tensile load of a polyethylene terephthalate (PET) film in each of machine and transverse directions is a value exceeding 3.0 N/10 mm even when the thickness thereof is as thin as 1.5 µm. Therefore, a patch obtained by using the PET film as the base layer is hard to closely adhere to the skin along the skin surface having fine irregularities such as skin furrows, and a stuck state is liable to be conspicuous. In addition, the patch obtained by using the PET film as the base layer gives a strong feeling of physical disorder upon sticking on the skin surface.

The elongation at break of the elastomer film used in the present invention in a machine direction (MD) is preferably 130 to 1,000%, more preferably 135 to 800%, particularly preferably 140 to 500%. Since the directional anisotropy of the elongation at break in the elastomer film is small, the elongation at break in a transverse direction (TD) is also at almost the same level as that in the machine direction.

If the elongation at break of the elastomer film is too small, the rigidity thereof becomes strong, and the stretchability thereof also becomes insufficient. Therefore, a patch obtained by using the elastomer film too small in elongation at break as the base layer is hard to closely adhere to the skin along the skin surface having fine irregularities such as skin furrows, a stuck state is liable to be conspicuous, and such a patch gives a strong feeling of physical disorder during sticking. If the elongation at break of the elastomer film is too great, there is a tendency for the stretchability of the resulting patch to become insufficient.

A colorant such as a pigment or dye may be contained in the elastomer film forming the base layer as needed. Various kinds of additives such as a stabilizer, an ultraviolet absorbent and a lubricant may also be contained in the elastomer film as needed.

### 4. Pressure sensitive adhesive layer

The pressure sensitive adhesive layer can be formed with a pressure sensitive adhesive exhibiting pressure sensitive adhesive property at ordinary temperature. In order to use the patch according to the present invention as a skin patch, it is preferable to use a pressure sensitive adhesive weak in skin irritation. Examples of the pressure sensitive adhesive include acrylic pressure sensitive adhesives, natural rubber-based pressure sensitive adhesives, synthetic rubber-based pressure sensitive adhesives, silicone-based pressure sensitive adhesives, vinyl ester-based pressure sensitive adhesives, vinyl ether-based pressure sensitive adhesives and urethane-based pressure sensitive adhesives. Among these, medical grade pressure sensitive adhesives are preferably used.

Among the pressure sensitive adhesives, acrylic pressure sensitive adhesives are preferred from the viewpoints of weak skin irritation, easy control of tackiness, excellent weather resistance and the like, and stable quality. As the acrylic pressure sensitive adhesives, are preferred copolymers obtained by using an alkyl acrylate or alkyl methacrylate, which is an esterified product of an aliphatic alcohol having 1 to 18 carbon atoms and acrylic acid or methacrylic acid. Specifically, as the acrylic pressure sensitive adhesives, are preferred copolymers of at least one acrylic monomer selected from the group consisting of an alkyl acrylate and an alkyl methacrylate each having an alkyl group having 1 to 18 carbon atoms, and another monomer copolymerizable with the acrylic monomer.

The acrylic pressure sensitive adhesives are preferably copolymers of 60 to 95% by weight of an alkyl acrylate having an alkyl group having 4 to 18 carbon atoms; 1 to 25% by weight of a vinyl monomer having at least one functional group selected from the group consisting of a hydroxyl group, a carboxyl group, an acid anhydride group, an amide group, an amino group, an epoxy group and an alkoxy group; and 0 to 40% by weight of another vinyl monomer copolymerizable with the alkyl acrylate. The alkyl acrylate is particularly preferably that having an alkyl group having 8 to 12 carbon atoms.

When the alkyl acrylate and/or alkyl methacrylate is represented as an alkyl (meth)acrylate, examples of the alkyl (meth)acrylate include methyl (meth)acrylate, ethyl (meth)acrylate, n-butyl (meth)acrylate, iso-butyl (meth)acrylate, t-butyl (meth)acrylate, n-hexyl (meth)acrylate, n-octyl (meth)acrylate, 2-ethylhexyl (meth)acrylate, iso-octyl (meth)acrylate, iso-nonyl (meth)acrylate, n-decyl (meth)acrylate, iso-decyl (meth)acrylate, lauryl (meth)acrylate and stearyl (meth)acrylate. These alkyl (meth)acrylates may be used either singly or in combination of 2 or more esters thereof.

Among these alkyl (meth)acrylates, are desired alkyl acrylates having 4 to 18 carbon atoms, preferably 8 to 12 carbon atoms, such as 2-ethylhexyl acrylate, n-octyl acrylate, iso-octyl acrylate and iso-nonyl acrylate.

Examples of the vinyl monomer having the functional group include acrylic esters having a hydroxyl group, such as 2-hydroxyethyl acrylate, 3-hydroxypropyl acrylate and 4-hydroxybutyl acrylate; vinyl monomers having a carboxyl group, such as acrylic acid, methacrylic acid, maleic acid, maleic anhydride, itaconic acid and monobutyl maleate; vinyl monomers having an amide group, such as acrylamide, dimethylacrylamide, diethylacrylamide, methacrylamide and N-methylolacrylamide; vinyl monomers having an amino group, such as dimethylaminoethyl acrylate; vinyl monomers having an epoxy group, such as glycidyl acrylate and glycidyl methacrylate; vinyl monomers having a pyrrolidone ring, such as N-vinylpyrrolidone; and alkoxyalkyl acrylates such as 2-methoxyethyl acrylate and ethoxyethyl acrylate. These monomers having the functional group may be used either singly or in combination of 2 or more monomers thereof.

Examples of another vinyl monomer copolymerizable with the alkyl acrylate include vinyl esters such as vinyl acetate; unsaturated nitriles such as acrylonitrile and methacrylonitrile; and vinyl aromatic compounds such as styrene.

When an alkyl acrylate having 4 to 18 carbon atoms, preferably 8 to 12 carbon atoms is used as the alkyl acrylate, any other alkyl (meth)acrylate may also be used as another vinyl monomer copolymerizable with the alkyl acrylate. As examples of said any other alkyl (meth)acrylate, may be mentioned alkyl acrylates such as methyl acrylate and ethyl acrylate; and alkyl methacrylates such as methyl methacrylate, ethyl methacrylate, n-butyl methacrylate, iso-butyl methacrylate, t-butyl methacrylate, n-hexyl methacrylate, n-octyl methacrylate, 2-ethylhexyl methacrylate, iso-octyl methacrylate, iso-nonyl methacrylate, n-decyl methacrylate, iso-decyl methacrylate, lauryl methacrylate and stearyl methacrylate.

The other vinyl monomers copolymerizable with the alkyl acrylate may be used either singly or in combination of 2 or more monomers thereof.

The acrylic pressure sensitive adhesive is desirably a copolymer of 60 to 95% by weight, preferably 65 to 95% by weight, more preferably 70 to 90% by weight of the alkyl acrylate having an alkyl group having 4 to 18 carbon atoms, preferably 8 to 12 carbon atoms; 1 to 25% by weight, preferably 1 to 20% by weight, more preferably 2 to 15% by weight of the vinyl monomer having the functional group; and 0 to 40% by weight, preferably 0 to 30% by weight, more preferably 0 to 25% by weight of said another vinyl monomer copolymerizable with the alkyl acrylate. The acrylic pressure sensitive adhesive having such a copolymerization composition is used, whereby a pressure sensitive adhesive layer exhibiting moderate tackiness even when the pressure sensitive adhesive layer is thin, and having excellent other properties can be easily formed.

The weight average molecular weight of the acrylic pressure sensitive adhesive is preferably 300,000 to 1,000,000, more preferably 450,000 to 650,000. The weight average molecular weight of the acrylic pressure sensitive adhesive is controlled within the above range, whereby cohesiveness, adhesive strength, workability upon mixing with other components, affinity for other components, and the like can be balanced with one another. The weight average molecular weight of the acrylic pressure sensitive adhesive is a value determined in terms of a value of standard polystyrene by gel permeation chromatography (GPC).

An acrylic ester copolymer can be generally synthesized by radical polymerization. Examples of a polymerization process include a solution polymerization process, an emulsion polymerization process and a bulk polymerization process. However, the solution polymerization process is preferred in that good tackiness is easily achieved. Examples of a polymerization initiator include organic peroxides such as benzoyl peroxide and lauroyl peroxide; and azo initiators such as azobisisobutyronitrile. The radical polymerization initiator is added in a proportion of about 0.1 to 3% by weight based on all the monomers, and the resultant mixture is stirred for from several hours to several tens hours at a temperature of about 40 to 90°C under a nitrogen atmosphere to conduct copolymerization. In the solution polymerization process, ethyl acetate, acetone, toluene, or a mixture thereof is commonly used as a solvent.

The thickness of the pressure sensitive adhesive layer is 1 to 9 µm, preferably 2 to 9 µm. In many cases, good results can be yielded when the thickness of the pressure sensitive adhesive layer falls within a range of from 2 to 8 µm, particularly from 2 to 7 µm.

In general, the pressure sensitive adhesive layer is small in resistance to stretching and high in stretchability compared with the base layer, so that the effects of the present invention can be achieved in a wider range than the thickness range of the base layer. However, if the thickness of the pressure sensitive adhesive layer is too great, there is a tendency to encounter difficulty in causing the resulting patch to closely adhere to the skin along the skin surface having fine irregularities such as skin furrows. In addition, the adhesive strength of the patch becomes too high, and there is a tendency for peeling after use to become difficult. If the thickness of the pressure sensitive adhesive layer is too thin, it is difficult to achieve moderate adhesive strength. In addition, it is difficult to cause the resulting patch to closely adhere to an adherend having fine irregularities, such as the skin, along the surface thereof.

Since the thickness of the pressure sensitive adhesive layer is thin, the adhesive strength of the pressure sensitive adhesive layer is preferably preset relatively high. However, the adhesive strength is more preferably preset within a moderate range from the viewpoint of balancing adhesion to the surface of an adherend such as the skin and easy peeling (releasability) after use with each other at a high level.

The pressure sensitive adhesive layer is required to exhibit adhesive strength of 0.1 N/10 mm or more in a 90-degree peeling test to a Bakelite plate prescribed in Japanese Industrial Standard JIS Z 0237. This adhesive strength is within a range of preferably from 0.1 to 3 N/10 mm, more preferably from 0.15 to 2 N/10 mm, particularly preferably from 0.15 to 1 N/10 mm. If the adhesive strength of the pressure sensitive adhesive layer is too low, the resulting patch is liable to be peeled by external force such as the movement of the skin when stuck on an adherend such as the skin. If the adhesive strength of the pressure sensitive adhesive layer is too strong, it is difficult to peel the patch from the skin of an adherend after use.

Although the lower limit value of the adhesive strength of the pressure sensitive adhesive layer of 0.1 N/10 mm is a very low value compared with the adhesive strength of a conventional patch, it has been proved that the adhesion as a patch is sufficient so far as the thickness and 10% tensile load value of the base layer satisfy the respective proper ranges because the base layer is thin and soft.

Various kinds of additives may be contained in the pressure sensitive adhesive layer as needed. In case of, for example, a skin patch, a percutaneously absorbable drug can be contained as an adhesive. Examples of the drug include drugs for angina, corticosteroidal drugs, analgesic-antiinflammatory drugs, antihistaminics, antibacterial drugs, moisturizers, vitamins and perfume bases. Various kinds of colorants such as pigments and dyes may be contained in the pressure sensitive adhesive layer.

### 5. Thickness of patch

In the patch according to the present invention, the thickness of the elastomer film forming the base layer is within a range of from 1 to 8 µm. The thickness of the pressure sensitive adhesive layer is 1 to 9 µm, preferably 2 to 9 µm.

The total thickness (may referred to as "the thickness of the patch" merely) of the base layer and the pressure sensitive adhesive layer is within a range of from 2 to 20 µm. The total thickness of the base layer and the pressure sensitive adhesive layer is within a range of preferably from 2 to 15 µm, more preferably from 3 to 14 µm. In many cases, good results can be yielded when the total thickness of the base layer and the pressure sensitive adhesive layer falls within an extremely thin range of from 3 to 10 µm or from 3 to 7 µm.

If the total thickness of the base layer and the pressure sensitive adhesive layer is too thin, the adhesive strength is lowered, or the production of the patch is difficult. If the total thickness of the base layer and the pressure sensitive adhesive layer is too great, the patch in a stuck state is liable to be conspicuous, and a feeling of physical disorder may be increased in some cases.

In the present invention, the thickness of each layer can be measured by a dial thickness gauge.

### 6. 10% Tensile load of patch

It is necessary that the 10% tensile load of the patch according to the present invention in each of machine and transverse directions is within a range of from 0.03 to 1.1 N/10 mm. The patch means a laminate (base layer/pressure sensitive adhesive layer) composed of 2 layers of the base layer and the pressure sensitive adhesive layer, excluding the carrier layer and the separator layer.

When the patch according to the present invention is used as a skin patch in particular, the patch can closely adhere to the skin along the skin surface having fine irregularities such as skin furrows and follow the movement of the skin. Such features relate to the degree of stretchability of the patch. In the present invention, a 10% tensile load value is adopted as an index indicating the degree of stretchability of the patch in view of usual expansion and contraction of the skin.

When the patch is stuck on the human skin, the patch is stretched in a plane direction upon sticking so far as the 10% tensile load of the patch in each of machine and transverse directions falls within a range of from 0.03 to 1.1 N/10 mm, and the patch is easy to closely adhere to the skin along the skin surface having fine irregularities such as skin furrows and can follow the usual movement of the skin.

The 10% tensile load of the patch is a value measured in accordance with Japanese Industrial Standard JIS Z 0237. The 10% tensile load value of the patch is within a range of from 0.03 to 1.1 N/10 mm, more preferably from 0.05 to 1.0 N/10 mm, particularly preferably from 0.06 to 0.95 N/10 mm in both machine and transverse directions.

If the 10% tensile load of the patch is too small, the thickness of the elastomer film as the base layer is generally too small, so that film-forming property and handling property are lowered. If the 10% tensile load of the patch is too great, the rigidity thereof becomes strong, and the stretchability and flexibility thereof are hence insufficient, so that such a patch is hard to closely adhere to the skin along the skin surface having fine irregularities such as skin furrows, and a stuck state is liable to be conspicuous. In addition, the patch too great in the 10% tensile load gives a strong feeling of physical disorder upon sticking on the skin surface.

In the patch according to the present invention, the 10% tensile load values are preferably substantially the same in both machine direction and transverse direction from the viewpoints of inconspicuousness of the patch in a stuck state and relief of a feeling of physical disorder.

### 7. Relationship between 10% tensile load and thickness of patch or base layer

Assuming that the 10% tensile load value of the patch according to the present invention in the machine direction (MD) is X (N/10 mm), the thickness value of the patch is Y (µm), and the thickness value of the base layer is Z (µm), the patch is required to satisfy the relationship that the product XY value of the 10% tensile load value X and the thickness value Y of the patch falls within a range of from 0.1 to 12, or the product XZ value of the 10% tensile load value X and the thickness value Z of the base layer falls within a range of from 0.05 to 6.8. Here, the patch means a 2-layer laminate composed of the base layer and the pressure sensitive adhesive layer.

The present inventors have found that the mere control of the material and thickness of the base layer, the thickness and adhesive strength of the pressure sensitive adhesive layer, the total thickness of the base layer and the pressure sensitive adhesive layer, and the 10% tensile load of the patch within the respective specific ranges is not sufficient to achieve inconspicuousness of the patch in a stuck state and relief of a feeling of physical disorder.

Thus, the present inventors have carried out a further investigation. As a result, it has been found that when the product XY value of the 10% tensile load value X of the patch in the machine direction and the thickness value Y of the patch, and/or the product XZ value of the 10% tensile load value X of the patch in the machine direction and the thickness value Z of the base layer are controlled so as to fall within the respective specific ranges, both inconspicuousness of the patch and relief of a feeling physical disorder can be sufficiently achieved. Since the 10% tensile load values of the patch in the machine and transverse directions are substantially not different, the 10% tensile load value in the machine direction is adopted in the present invention.

When the thickness of the patch or base layer is relatively great even when the 10% tensile load value of the patch is relatively small, such a patch is hard to closely adhere to the skin along the skin surface having fine irregularities such as skin furrows and to become a state that a fine texture structure of the skin surface has been transferred to the back surface through the patch. As a result, the patch in a stuck state is liable to be conspicuous.

When the 10% tensile load value of the patch is relatively great even when the thickness of the patch or base layer is relatively small, such a patch is hard to closely adhere to the skin along the skin surface having fine irregularities such as skin furrows and to become a state that a fine texture structure of the skin surface has been transferred to the back surface through the patch. As a result, the patch in a stuck state is liable to be conspicuous.

If the XY value and/or the XZ value is too great, the patch in a stuck state is liable to be conspicuous, and there is also a tendency for a feeling of physical disorder to increase.

The present inventors have arranged experimental data as to the relationship between the 10% tensile load value of the base layer, the thickness of the patch and the thickness of the base layer, and the inconspicuousness of the patch and the feeling of physical disorder. As a result, it has been found that when the product XY value of the 10% tensile load value X of the patch and the thickness value Y of the patch falls within a range of from 0.1 to 12, and/or the product XZ value of the 10% tensile load value X of the patch and the thickness value Z of the base layer falls within a range of from 0.05 to 6.8, both inconspicuousness of the patch and relief of the feeling of physical disorder can be balanced with each other at a high level in cooperation with the requirements of the thickness of each layer, the adhesive strength and the like.

FIG. 1 illustrates a conceptual diagram of a region represented by the product XY value of the 10% tensile load value X of a patch and the thickness value Y of the patch. The patch according to the present invention falls within a rectangle indicated by the upper and lower limit values of the thickness of the patch, and the upper and lower limit values of the 10% tensile load value as illustrated in FIG. 1. However, it has been proved that when the experimental data are arranged, there is a region, in which the inconspicuousness of the patch and the relief of the feeling of physical disorder are not sufficiently achieved even when the patch falls within the region of the rectangle illustrated in FIG. 1. This region is a region indicated by B in FIG. 1.

The patch according to the present invention falls within a region A (X = 0.01 to 1.2 N/10 mm, Y = 2 to 20 µm, XY = 0.02 to 15) in the rectangular region illustrated in FIG. 1. The relationship between the 10% tensile load value X of the patch and the thickness Z of the base layer is the same as in FIG. 1. The 10% tensile load of the patch is greatly affected by the base layer and substantially almost consists with the 10% tensile load value of the base layer. For example, when the thickness of the pressure sensitive adhesive layer is changed with the thickness of the base layer fixed, or the thickness of the base layer is changed with the thickness of the pressure sensitive adhesive layer fixed, a change in the former is smaller than the latter. Thus, the region A in the patch is in a form laterally longer than the region defined by the thickness of the base layer.

The product XY value of the 10% tensile load X (N/10 mm) of the patch according to the present invention in the machine direction and the thickness Y (µm) of the patch is within a range of from 0.1 to 12, more preferably from 0.3 to 11.

The product XZ value of the 10% tensile load X (N/10 mm) of the patch according to the present invention in the machine direction and the thickness Z (µm) of the base layer is within a range of from 0.05 to 6.8, more preferably from 0.08 to 6.5.

If the XY value and/or the XZ value is too great, the patch in a stuck state is liable to be conspicuous, and the feeling of physical disorder may also be increased in many cases. If the XY value and/or the XZ value is too small, other properties such as adhesive strength are lowered, or the production of the patch is difficult.

### 8. Touch of Patch

The touch of the surface (back surface of the base layer) of the patch according to the present invention can be determined by the slip property of the surface. In order to improve the slip property of the surface of the patch, fine irregularities are preferably provided on the back surface of the base layer. It is thus preferable to adopt a process, in which a carrier layer is finely embossed, and a base layer is formed thereon, thereby forming fine irregularities on the back surface of the base layer.

The slip property can be quantified by a coefficient of dynamic friction. The coefficient of dynamic friction can be measured by the following method with the horizontal method prescribed in Japanese Industrial Standard JIS P 8147 partly changed.

A maximum load of a load cell of a constant-rate-of-extension tensile tester is determined to be 10 N, and a possibly flat place of a forearm is used in place of a horizontal plate. A brass-made cube 20 mm square is used as a weight. The patch according to the present invention is stuck on one surface of this weight so as not to flaw or wrinkle, the stuck surface is placed on the forearm, and a hook fitted to the weight through a fine metal wire is pulled. A pulley is fixed to a pendent portion of the load cell in advance and located at just above the wrist, whereby the weight can be horizontally moved. The moving speed and moving distance of the weight are determined to be 100 mm/min and 50 mm, respectively, and friction force therebetween is recorded. The friction force recorded while the weight is being moved is regarded as dynamic friction force. A value obtained by dividing the resultant average dynamic friction force by a vertical load by the weight is regarded as a coefficient of dynamic friction.

When the coefficient of dynamic friction as measured by the above-described method is 1.0 or less, a feeling of physical disorder in the slip property of the surface of the patch to the skin is lost. When the surface of the patch is embossed in such a manner that the coefficient of dynamic friction is 1.0 or less, the gloss of the surface of the patch is moderately lowered, and so the patch becomes inconspicuous even from the viewpoint of appearance. The lower limit value of the coefficient of dynamic friction is generally 0.1.

### 9. Other properties of patch

In the patch according to the present invention, the ultraviolet transmittance in a wavelength range of from 280 to 400 nm can be controlled to preferably 25% or less, more preferably 15% or less, still more preferably 10% or less. Here, the patch means a 2-layer laminate of the base layer and the pressure sensitive adhesive layer.

The ultraviolet transmittance may become a problem according to uses of the patch. When the patch according to the present invention is used as, for example, a medical patch, it may be desirable in some cases to protect a damaged site from ultraviolet rays. The patch according to the present invention can exhibit good ultraviolet-screening property without causing an ultraviolet absorbent to be contained therein. A method, in which an ultraviolet absorbent is caused to be contained in the base layer and/or the pressure sensitive adhesive layer, can be adopted for more reducing the ultraviolet transmittance of the patch according to the present invention.

The base layer and/or the pressure sensitive adhesive layer may be preferably colored according to uses of the patch. The skin patch may be colored into, for example, a human skin color for making a stuck state inconspicuous. Various kinds of colorants such as pigments, dyes and inks may be used in coloring.

In the patch according to the present invention, the water vapor transmission rate can be controlled to 1,000 g/m²·24 hr or more, preferably 2,000 g/m²·24 hr or more, more preferably 2,500 g/m²·24 hr or more, particularly preferably 3,000 g/m²·24 hr or more. The upper limit value of the water vapor transmission rate is generally 10,000 g/m²·24 hr, often 5,000 g/m²·24 hr. The water vapor transmission rate is a value measured under the conditions B (temperature: 40°C, relative humidity: 90%) prescribed in Japanese Industrial Standard JIS Z 0208. The high water vapor transmission rate of the patch can prevent occurrence of stuffiness or accumulation of sweat during sticking.

### 10. Carrier layer

Since the patch according to the present invention is extremely thin and rich in stretchability, its handling property is lowered in addition to difficulty in forming the base layer when the patch does not contain a carrier layer. When the patch according to the present invention does not contain the carrier layer, the patch is hard to be successfully stuck on an adherend, and in some cases, the base layer may wrinkle, or pressure sensitive adhesive layers may stick on each other. The base layer is temporarily stuck on the carrier layer, whereby the ability to form the base layer, and the handling property and the sticking property on the adherend of the patch can be improved. Since the carrier layer is provided for improving the handling property of the patch, it may be covered on the whole surface of the patch, only on edge portions of the patch, or in a patterned form such as grating.

The carrier layer is preferably formed with a film composed of any one of various kinds of thermoplastic resins, for example, polyurethane, polyethylene, polypropylene, ionomers, polyamide, polyvinyl chloride, polyvinylidene chloride, ethylene-vinyl acetate copolymers, thermoplastic polyesters and polytetrafluoroethylene.

Aiming at environmental protection, the carrier layer may also be formed with a film composed of any one of various kinds of plastics having biodegradability, typified by polyhydroxybutyrate, polyhydroxybutyrate resins, polyhydroxyalkanoates, maltotriose, polylactic acid, polylactic acid resins, polyethylene succinate, polyethylene succinate resins, polybutylene succinate resins, polycaprolactone resins, poly(butylene adipate-co-terephthalate), poly(tetramethylene adipate-co-terephthalate), poly(ethylene terephthalate) resins, polyvinyl alcohol, polyglycolic acid, starch fatty acid esters, starch-processed resins, starch polyesters, cellulose acetate and chitosan.

The various kinds of films may be in a state laminated on paper. These carrier layers desirably have a thicker thickness or stronger stiffness compared with the polyurethane elastomer layer. The thickness of the carrier layer may be suitably set. However, it is generally 10 µm or more, preferably 20 µm or more, and the upper limit value thereof is about 500 µm.

Since the elastomer film of the base layer is firmly bonded to the pressure sensitive adhesive layer, the carrier layer can be easily peeled upon use.

### 11. Separator layer

In the patch, a separator layer is preferably provided from the viewpoint of protecting the pressure sensitive adhesive layer till just before sticking. As the separator layer, may be used one generally called lubricant paper, lubricant film, release paper, release film, release liner or the like in a technical field of pressure sensitive adhesive tapes. As representative examples of the separator layer, may be mentioned polyethylene terephthalate films, the surfaces of which have been subjected to a silicone treatment, and laminates of polyethylene, the surface of which has been subjected to a silicone treatment, and paper.

### EXAMPLES

The present invention will hereinafter be described more specifically by Examples and Comparative Examples. However, the present invention is not limited to these examples.

The measuring methods and evaluating methods in the present invention are as follows.

### (1) 10% Tensile load

A 10% tensile load of a patch was measured in accordance with Japanese Industrial Standard JIS Z 0237. Specifically, the patch was stretched by 10% by an Instron type tensile tester to measure a load (N) at that time. The resultant value was converted to a value in a 10-mm width. The measurement was conducted in both machine and transverse directions. However, the measured values were substantially the same, and so the measured value in the machine direction was adopted. The 10% tensile load of a base layer was measured by the same method.

### (2) Evaluation of inconspicuousness

Specimens obtained by cutting a patch in a size of 17 mm x 29 mm were used to evaluate the degree of inconspicuousness when stuck on the human skin. The specimens were stuck on the backs of the hands and forearms of 5 adult men and women. The stuck state was evaluated visually and by an image through a confocal microscope obtained by using a model with the state stuck on the skin surface transferred to an impression material for dental surgery. The evaluation standard is as follows.
AA: The stuck states on 4 or 5 subjects were hard to be conspicuous.
A: The stuck states on 3 subjects were hard to be conspicuous.
B: The stuck states on 2 subjects were hard to be conspicuous.
C: The stuck state one or zero subject was hard to be conspicuous.

### (3) Evaluation of a feeling of physical disorder

Evaluation of a feeling of physical disorder was inquired of the 5 subjects responded to the above test (2) to rank it in accordance with the following standard.
AA: 4 or 5 subjects felt no feeling of physical disorder.
A: 3 subjects felt no feeling of physical disorder.
B: 2 subjects felt no feeling of physical disorder.
C: One or zero subject felt no feeling of physical disorder.

### (4) Evaluation of adhesion

A patch was used to conduct a 90-degree peeling test to a Bakelite plate as prescribed in Japanese Industrial Standard JIS Z 0237, thereby measuring adhesive strength (peeling force). Specifically, a patch 10-mm wide was stuck on a Bakelite plate to measure adhesive strength (N) at the time the patch was peeled in a direction of 90° by an Instron type tensile tester. The adhesion was evaluated in accordance with the following standard based on this measured value of the adhesive strength. This adhesion is considered to consist with evaluation of the adhesion to the skin.
A: The adhesive strength was 0.1 N/10 mm or more.
B: The adhesive strength was less than 0.1 N/10 mm.

### (5) Elongation at break

The elongation at break of an elastomer film was measured by the following method. An elastomer film was cut into a width of 20 mm along a machine direction (MD) and then cut into a length of about 75 mm to prepare a specimen. This specimen was fitted to an Instron type tensile tester (using load cells of 10 N and 100 N) with a distance between chucks set to 50 mm. The specimen was pulled at a moving speed of 300 mm/min. Elongation at the time the specimen was broken was read to calculate an elongation (%) at break.

### (6) Ease of peeling

Specimens obtained by cutting a patch in a size of 17 mm x 29 mm were stuck on the backs of the hands and forearms of 5 adult men and women. After 30 minutes, the specimens were peeled from the stuck sites. Ease of peeling was evaluated in accordance with the following standard.
AA: The specimen can be easily peeled from an end portion.
A: An end portion for peeling is hard to be gripped.
B: An end portion for peeling is hard to be gripped, and moreover the patch may be broken during peeling in some cases.

### (7) Water vapor transmission rate

A water vapor transmission rate of a patch was measured under the conditions B (temperature: 40°C, relative humidity: 90%) prescribed in Japanese Industrial Standard JIS Z 0208.

### (8) Coefficient of dynamic friction

A coefficient of dynamic friction was measured by the above-described method with the horizontal method prescribed in Japanese Industrial Standard JIS P 8147 partly changed. Subjects for measurement of the coefficient of dynamic friction were 10 adult men and women.

### [Example 1]

An organic solvent solution of an acrylic pressure sensitive adhesive (a copolymer of 2-ethylhexyl acrylate/ vinyl acetate/acrylic acid = 85/11/4 weight ratio) was applied to one surface of a separator layer (release paper) by a bar coating method so as to give a dry coating thickness of 5 µm and then dried to form a pressure sensitive adhesive layer.

Woodfree paper, on which a polyethylene had been laminated, was used as a carrier layer, and a solution of an ether type water-vapor-permeable polyurethane elastomer was applied on to the surface on the polyethylene side by a bar coating method so as to give a dry coating thickness of 5 µm and dried to form a base layer. The solution of the ether type polyurethane elastomer is "LUCKSKIN US2268" (trade name; glass transition temperature: -23.1°C) available from SEIKOH CHEMICALS CO., LTD.

The base layer obtained above was laminated on the pressure sensitive adhesive layer to produce a patch having a laminated structure of carrier layer/base layer/pressure sensitive adhesive layer/separator layer. In this patch, the separator layer could be peeled upon use, and the carrier layer could be peeled after stuck on the skin. The patch in a stuck state closely adhered to the skin along the skin surface having fine irregularities such as skin furrows and did not give a feeling of physical disorder.

This patch was such that the thickness Y of the patch is 10 µm, the thickness of the base layer is 5 µm, the elongation at break of the base layer is 356%, the thickness of the pressure sensitive adhesive layer is 5 µm, the 10% tensile load in each of machine and transverse directions is 0.15 N/10 mm, the XY value is 1.50, the XZ value is 0.75, the adhesive strength of the pressure sensitive adhesive layer is 0.59 N/10 mm, and the water vapor transmission rate is 3,280 g/m²·24 hr.

Accordingly, this patch was inconspicuous in a stuck state, did not give a feeling of physical disorder, was excellent in adhesion and had sufficient air permeability.

The ultraviolet transmittance of this patch within a wavelength range of from 280 to 400 nm was as illustrated in FIG. 4.

### [Example 2]

Woodfree paper, the polyethylene surface of which had been embossed, was used as a carrier layer, the polyurethane elastomer solution used in Example 1 was applied on to the surface on the polyethylene side by a bar coating method so as to give a dry coating thickness of 5 µm in the same manner as in Example 1 and dried to form a base layer, and the base layer and a pressure sensitive adhesive layer were then laminated to produce a patch.

In this patch, an embossed pattern was transferred to the surface of the base layer by the embossed pattern subjected to the surface on the polyethylene side of the carrier layer, and this embossed pattern made the patch more inconspicuous when the patch was stuck on the skin. The embossed pattern provided a patch apparently free of a feeling of physical disorder between the skin and the patch. With respect to this patch, the coefficient of dynamic friction was determined on 10 adult men and women by the changed method of JIS P 8147. As a result, it was within a range of from 0.35 to 0.80. The slip property of the patch to the skin was judged to be good by all the subjects. Other properties were the same as in the patch of Example 1. The results are shown in Table 1.

### [Examples 3 to 9, and Comparative Examples 1 to 6]

Patches were produced in the same manner as in Example 2 except that US2268 (Tg = -23.1°C), U-1223 (Tg = -29.0°C), U-1285, U-2860, and U-1285/U-1223 (9:1 mixture) of LUCKSKIN (trademark) series available from SEIKOH CHEMICALS CO., LTD. were respectively used as the polyurethane elastomer solution as shown in Table 1, and the thickness of the base layer and the thickness of the pressure sensitive adhesive layer were changed. The results are shown in Table 1.

FIG. 2 illustrates an image through a confocal microscope obtained by sticking a patch (in which the thickness of the base layer was 1 µm, and the thickness of the pressure sensitive adhesive layer was 2 µm) prepared in Example 4 on the surface of the human skin, and using a model obtained by transferring the stuck state to an impression material for dental surgery. A left half of FIG. 2 shows a back surface of the patch, and a right half thereof shows the fine structure of the skin surface.

As apparent from FIG. 2, the patch according to the present invention closely adheres to the skin along the skin surface having fine irregularities such as skin furrows when stuck on the surface of the human skin in a state as if a fine texture structure of the skin surface would be transferred to a back surface of the patch through the patch, and so the stuck portion is hard to be conspicuous.

FIG. 3 illustrates an image through a confocal microscope obtained by sticking a patch (in which the thickness of the base layer was 18 µm, and the thickness of the pressure sensitive adhesive layer was 7 µm) prepared in Comparative Example 4 on the surface of the human skin, and using a model obtained by transferring the stuck state to an impression material for dental surgery. A left half of FIG. 3 shows a back surface of the patch, and a right half thereof shows the fine structure of the skin surface.

As apparent from Table 3, the patch of Comparative Example 4 does not satisfy the requirements defined in claim 1 of the present invention, so that the patch does not closely adhere to the finely irregular surface of the skin in a stuck state and becomes a state as if it would rise above the skin, and the fine texture structure on the skin surface is not in the state transferred to the back surface of the patch, and so the stuck portion is liable to be conspicuous.

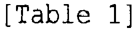

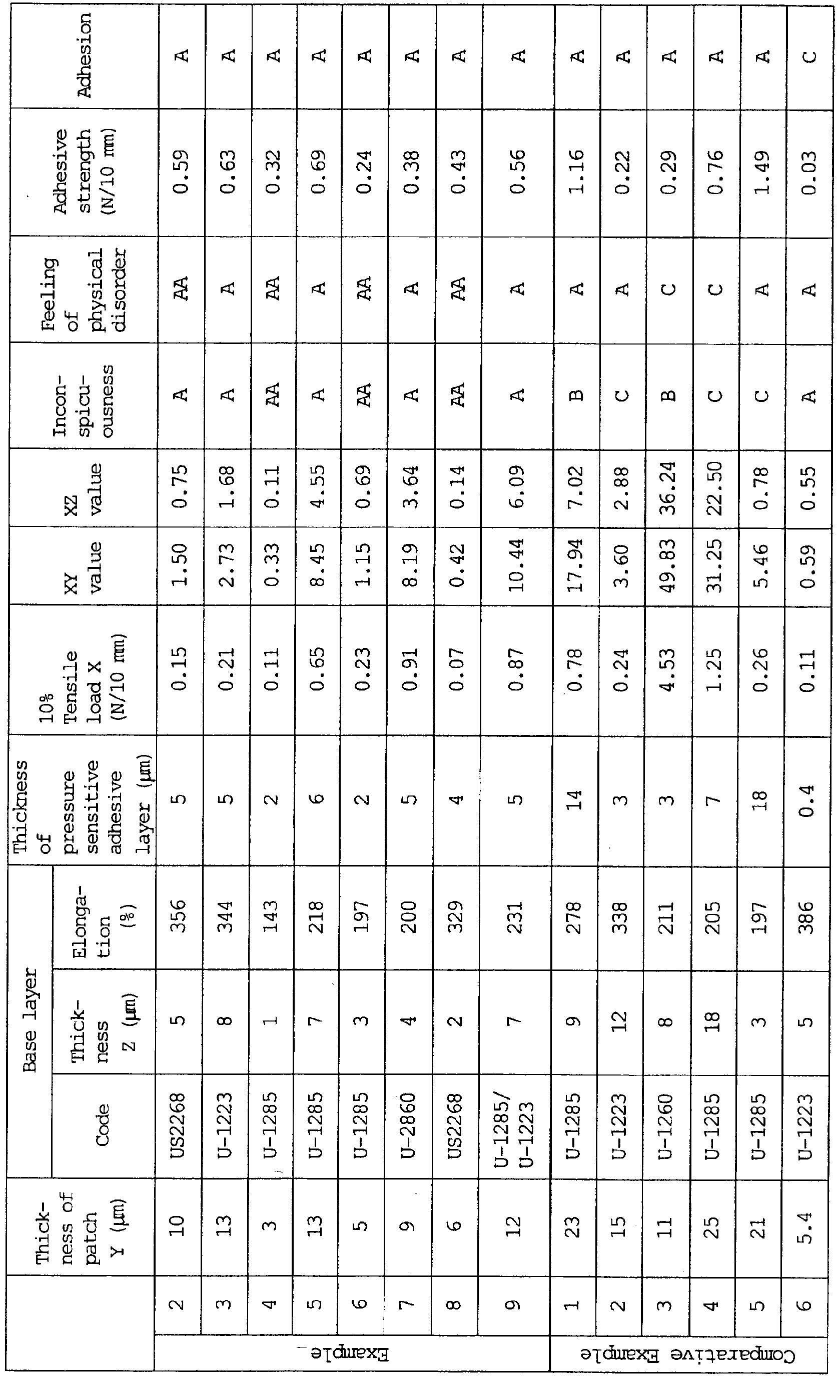

### (Consideration)

The patches of Examples 2 to 9 are such that the stuck portion of the patch is scarcely conspicuous, no feeling of physical disorder is given, and adhesion is good.

On the other hand, the patches of Comparative Examples 1 to 5 are such that a stuck portion is conspicuous, or a feeling of physical disorder is given because the patches do not satisfy the requirements defined in claim 1 of the present invention though adhesion is good. The patch of Comparative Example 6 is insufficient in adhesive strength.

### [Examples 10 to 17, and Comparative Examples 7 and 8]

Various patches were produced in the same manner as in Example 1 except that the thickness of the pressure sensitive adhesive layer was changed. The results are shown in Table 2.

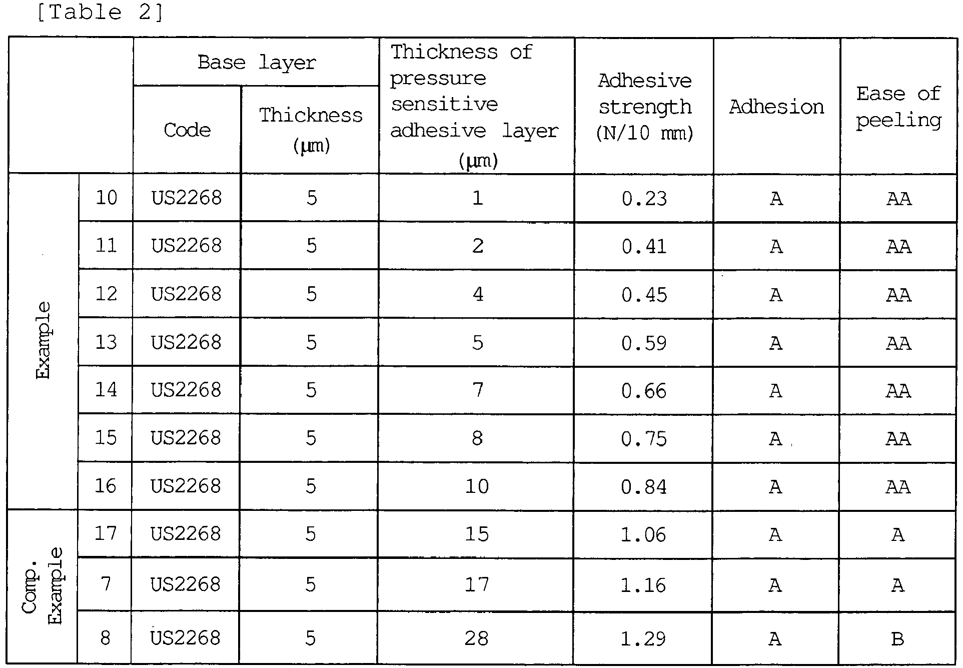

As apparent from the results shown in Table 2, it is understood that ease of peeling from the skin surface is lowered as the thickness of the pressure sensitive adhesive layer increases, or the adhesive strength becomes great.

### [Comparative Examples 9 to 11]

Three patches different in the thickness of the base layer were produced in the same manner as in Example 1 except that polyethylene terephthalate (PET) was used in place of the polyurethane elastomer. The results are shown in Table 3.

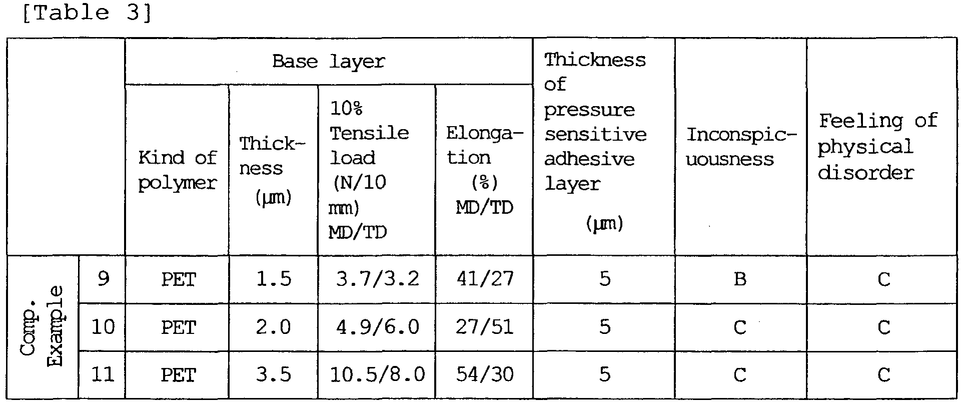

As apparent from the results shown in Table 3, the patches are such that the stuck portion of the patch is liable to be conspicuous, and a feeling of physical disorder is given even when the thickness of the PET film is thinned. In addition, the base layer composed of the PET film was small in elongation, so that the base layer was broken during sticking, and good tackiness was not achieved.

### INDUSTRIAL APPLICABILITY

The patches according to the present invention can closely adhere to an adherend along the surface having fine irregularities, so that the patches in a stuck state are inconspicuous. In addition, the patches according to the present invention does not give a feeling of physical disorder when used as skin patches.

Accordingly, the patches according to the present invention can be used in a wide variety of fields such as medical patches, first-aid adhesive bandages, waterproofing patches and protecting patches for protecting the skin. In addition, the patches according to the present invention can be utilized as patches in a wide variety of fields of industries, utensils, household and the like.

## Claims

1. A patch having a layer structure in which a pressure sensitive adhesive layer is provided on one surface of a base layer, wherein
(a) the base layer is a polyurethane elastomer film having a thickness with a range of from 1 to 8 µm,
(b) the thickness of the pressure sensitive adhesive layer is within a range of from 1 to 9 µm,
(c) the total thickness of the base layer and the pressure sensitive adhesive layer is within a range of from 2 to 15 µm,
(d) the 10% tensile load of the patch in each of machine and transverse directions is within a range of from 0.03 to 1.1 N/10 mm as measure in accordance with Japanese Industrial Standard JIS Z 0237,
(e) assuming that the 10% tensile load value of the patch in the machine direction is X (N/10 mm), the thickness value of the patch is Y (µm), and the thickness value of the base layer is Z (µm), the patch satisfies the relationship that the product XY value of the 10% tensile load value X and the thickness value Y of the patch falls within a range of from 0.1 to 12, or the product XZ value of the 10% tensile load value X and the thickness value Z of the base layer falls within a range of from 0.05 to 6.8,
(f) the pressure sensitive adhesive layer exhibits adhesive strength of 0.15 to 2 N/10 mm in a 90-degree peeling test to a Bakelite plate prescribed in Japanese Industrial Standard JIS Z 0237, and
(g) the pressure sensitive adhesive layer is any one selected from the group consisting of acrylic pressure sensitive adhesive layer, natural rubber-based pressure sensitive adhesive layer, synthetic rubber-based pressure sensitive adhesive layer, silicone-based pressure sensitive adhesive layer, vinyl ester-based pressure sensitive adhesive layer, vinyl ether-based pressure sensitive adhesive layer and urethane-based pressure sensitive adhesive layer.

2. The patch according to claim 1, wherein the polyurethane elastomer is a polyether type polyurethane elastomer or polyester type polyurethane elastomer.

3. The patch according to claim 1, wherein the pressure sensitive adhesive layer is an acrylic pressure sensitive adhesive layer.

4. The patch according to claim 3, wherein the acrylic pressure sensitive adhesive is a copolymer of at least one acrylic monomer selected from the group consisting of an alkyl acrylate and an alkyl methacrylate each having an alkyl group having 1 to 18 carbon atoms, and another monomer copolymerizable with the acrylic monomer.

5. The patch according to claim 3, wherein the acrylic pressure sensitive adhesive is a copolymer of 60 to 95% by weight of an alkyl acrylate having an alkyl group having 4 to 18 carbon atoms; 1 to 25% by weight of a vinyl monomer having at least one functional group selected from the group consisting of a hydroxyl group, a carboxyl group, an acid anhydride group, an amide group, an amino group, an epoxy group and an alkoxy group; and 0 to 40% by weight of another vinyl monomer copolymerizable with the alkyl acrylate.

6. The patch according to claim 1, wherein the elongation at break of the elastomer film in a machine direction is within a range of from 130 to 1000% as measured in accordance with the method under the heading "Elongation at Break" in the description.

7. The patch according to claim 1, wherein the glass transition temperature of an elastomer forming the elastomer film is within a range of from -70°C to 20°C.

8. The patch according to claim 1, wherein the water vapor transmission rate of the patch is 1000 g/m²·24 hr or more as measured in accordance with Japanese Industrial Standard JIS Z 0208 under the conditions of 40 °C of temperature and 90% of relative humidity.

9. The patch according to claim 1, wherein at least one of the base layer and the pressure sensitive adhesive layer contains a colorant.

10. The patch according to claim 1, wherein the ultraviolet transmittance of the patch within a wavelength range of from 280 to 400 nm is 25% or less.

11. The patch according to claim 1, which has a laminated structure of "carrier layer/base layer/pressure sensitive adhesive layer/separator layer", in which a carrier layer is arranged on the surface of the base layer on the side reversed to the pressure sensitive adhesive layer, and a separator layer is arranged on the surface of the pressure sensitive adhesive layer on the side reversed to the base layer.

12. The patch according to claim 1, wherein the surface of the base layer on the side reversed to the pressure sensitive adhesive layer is embossed.

## Patentansprüche

1. Pflaster mit einer Schichtstruktur, in dem eine druckempfindliche Klebeschicht auf einer Oberfläche einer Basisschicht bereitgestellt wird, wobei
(a) die Basisschicht eine Polyurethan-Elastomer-Folie ist, die eine Dicke mit einem Bereich von 1 bis 8 µm aufweist,
(b) die Dicke der druckempfindlichen Klebeschicht in einem Bereich von 1 bis 9 µm liegt,
(c) die Gesamtdicke der Basisschicht und der druckempfindlichen Klebeschicht in einem Bereich von 2 bis 15 µm liegt,
(d) die 10%ige Zugbelastung des Pflasters in jeweils den Längs- und Querrichtungen, gemessen gemäß der Japanischen Industrienorm JIS Z 0237, in einem Bereich von 0,03 bis 1,1 N/10 mm liegt,
(e) mit der Maßgabe, dass der 10%ige Zugbelastungswert des Pflasters in der Längsrichtung X (N/10 mm) ist, der Dickenwert des Pflasters Y (µm) ist und der Dickenwert der Basisschicht Z (µm) ist, das Pflaster die Beziehung erfüllt, dass der Produktwert XY des 10%igen Zugbelastungswerts X und des Dickenwerts Y des Pflasters in einen Bereich von 0,1 bis 12 fällt, oder der Produktwert XZ des 10%igen Zugbelastungswerts X und des Dickenwerts Z der Basisschicht in einen Bereich von 0,05 bis 6,8 fällt,
(f) die druckempfindliche Klebeschicht eine Klebefestigkeit von 0,15 bis 2 N/10 mm in einem in der Japanischen Industrienorm JIS Z 0237 beschriebenen 90-Grad-Abziehversuch an einer Bakelitplatte aufweist, und
(g) die druckempfindliche Klebeschicht jedwede eine aus der Gruppe ausgewählte ist, bestehend aus einer druckempfindlichen Acrylklebeschicht, einer auf Naturkautschuk basierenden druckempfindlichen Klebeschicht, einer auf synthetischem Kautschuk basierenden druckempfindlichen Klebeschicht, einer auf Silikon basierenden druckempfindlichen Klebeschicht, einer auf Vinylester basierenden druckempfindlichen Klebeschicht, einer auf Vinylether basierenden druckempfindlichen Klebeschicht und einer auf Urethan basierenden druckempfindlichen Klebeschicht.

2. Pflaster nach Anspruch 1, wobei das Polyurethan-Elastomer ein Polyurethan-Elastomer des Polyethertyps oder ein Polyurethan-Elastomer des Polyestertyps ist.

3. Pflaster nach Anspruch 1, wobei die druckempfindliche Klebeschicht eine druckempfindliche Acrylklebeschicht ist.

4. Pflaster nach Anspruch 3, wobei die druckempfindliche Acrylklebeschicht Folgendes ist: ein Copolymer von mindestens einem aus der Gruppe ausgewählten Acrylmonomer, bestehend aus einem Alkylacrylat und einem Alkylmethacrylat, wobei jedes eine Alkylgruppe mit 1 bis 18 Kohlenstoffatom(en) aufweist, und ein anderes mit dem Acrylmonomer copolymerisierbares Monomer.

5. Pflaster nach Anspruch 3, wobei der druckempfindliche Acrylklebstoff Folgendes ist: ein Copolymer von 60 bis 95 Gew.-% eines Alkylacrylats mit einer Alkylgruppe, die 4 bis 18 Kohlenstoffatome aufweist; 1 bis 25 Gew.-% eines Vinylmonomers, das mindestens eine funktionelle Gruppe aufweist, die aus der Gruppe ausgewählt ist, bestehend aus einer Hydroxylgruppe, einer Carboxylgruppe, einer Säureanhydridgruppe, einer Amidgruppe, einer Aminogruppe, einer Epoxygruppe und einer Alkoxygruppe; und 0 bis 40 Gew.-% eines anderen mit dem Alkylacrylat copolymerisierbaren Vinylmonomers.

6. Pflaster nach Anspruch 1, wobei die Dehnung beim Reißen der Elastomer-Folie in einer Längsrichtung, wie gemäß dem Verfahren unter der Überschrift "Dehnung beim Reißen" in der Beschreibung gemessen, in einem Bereich von 130 bis 1000 % liegt.

7. Pflaster nach Anspruch 1, wobei die Glasübergangstemperatur eines Elastomers, das die Elastomer-Folie bildet, in einem Bereich von -70 °C bis 20 °C liegt.

8. Pflaster nach Anspruch 1, wobei die Wasserdampfdurchlässigkeitsrate des Pflasters, wie gemäß der Japanischen Industrienorm JIS Z 0208 unter den Bedingungen einer Temperatur von 40 °C und einer relativen Feuchte von 90 % gemessen, 1000 g/m²·24 h oder mehr beträgt.

9. Pflaster nach Anspruch 1, wobei mindestens eine der Basisschicht und der druckempfindlichen Klebeschicht einen Farbstoff enthält.

10. Pflaster nach Anspruch 1, wobei die Ultraviolettdurchlässigkeit des Pflasters in einem Wellenlängenbereich von 280 bis 400 nm 25 % oder weniger liegt.

11. Pflaster nach Anspruch 1, das eine laminierte Struktur aus der "Trägerschicht/Basisschicht/druckempfindlichen Klebeschicht/Trennschicht" aufweist, wobei eine Trägerschicht auf der Oberfläche der Basisschicht auf der Rückseite der druckempfindlichen Klebeschicht angeordnet ist, und eine Trennschicht auf der Oberfläche der druckempfindlichen Klebeschicht auf der Rückseite der Basisschicht angeordnet ist.

12. Pflaster nach Anspruch 1, wobei die Oberfläche der Basisschicht auf der Rückseite der druckempfindlichen Klebeschicht geprägt ist.

## Revendications

1. Un timbre ayant une structure en couches dans lequel une couche d'adhésif sensible à la pression est fournie sur une surface d'une couche de base, où
(a) la couche de base est un film élastomère de polyuréthane ayant une épaisseur comprise entre 1 et 8 µm,
(b) l'épaisseur de la couche d'adhésif sensible à la pression est comprise entre 1 et 9 µm,
(c) l'épaisseur totale de la couche de base et de la couche d'adhésif sensible à la pression est comprise entre 2 et 15 µm,
(d) l'effort de traction de 10% du timbre dans chacun des sens machine et transversal est compris entre 0,03 et 1,1 N/10 mm tel que mesuré selon la norme industrielle japonaise JIS Z 0237,
(e) en partant de l'hypothèse que la valeur de l'effort de traction de 10% du timbre dans le sens machine est X (N/10 mm), que la valeur de l'épaisseur du timbre est Y (µm) et que la valeur de l'épaisseur de la couche de base est Z (µm), le timbre satisfait la relation que la valeur XY du produit de la valeur d'effort de traction X de 10% et de la valeur d'épaisseur Y du timbre est comprise entre 0,1 et 12, ou la valeur XZ du produit de la valeur d'effort de traction X de 10% et de la valeur d'épaisseur Z de la couche de base est comprise entre 0,05 et 6,8,
(f) la couche d'adhésif sensible à la pression présente un pouvoir d'adhérence de 0,15 à 2 N/10 mm dans un essai de pelage à 90 degrés d'une plaque de Bakelite, prescrit dans la norme industrielle japonaise JIS Z 0237, et
(g) la couche d'adhésif sensible à la pression est l'une quelconque sélectionnée dans le groupe constitué par une couche d'adhésif acrylique sensible à la pression, une couche d'adhésif sensible à la pression à base de caoutchouc naturel, une couche d'adhésif sensible à la pression à base de caoutchouc synthétique, une couche d'adhésif sensible à la pression à base de silicone, une couche d'adhésif sensible à la pression à base d'ester vinylique, une couche d'adhésif sensible à la pression à base d'éther vinylique et une couche d'adhésif sensible à la pression à base d'uréthane.

2. Le timbre selon la revendication 1, où l'élastomère de polyuréthane est un élastomère de polyuréthane de type polyéther ou un élastomère de polyuréthane de type polyester.

3. Le timbre selon la revendication 1, où la couche d'adhésif sensible à la pression est une couche d'adhésif acrylique sensible à la pression.

4. Le timbre selon la revendication 3, où l'adhésif acrylique sensible à la pression est un copolymère d'au moins un monomère acrylique sélectionné dans le groupe constitué par un acrylate d'alkyle et un méthacrylate d'alkyle, chacun ayant un groupe alkyle ayant 1 à 18 atomes de carbone, et un autre monomère copolymérisable avec le monomère acrylique.

5. Le timbre selon la revendication 3, où l'adhésif acrylique sensible à la pression est un copolymère de 60 à 95% en poids d'un acrylate d'alkyle ayant un groupe alkyle ayant 4 à 18 atomes de carbone ; 1 à 25% en poids d'un monomère vinylique ayant au moins un groupe fonctionnel sélectionné dans le groupe constitué par un groupe hydroxyle, un groupe carboxyle, un groupe anhydride d'acide, un groupe amide, un groupe amino, un groupe époxy et un groupe alkoxy ; et 0 à 40% en poids d'un autre monomère vinylique copolymérisable avec l'acrylate d'alkyle.

6. Le timbre selon la revendication 1, où l'allongement à la rupture du film élastomère dans un sens machine est compris entre 130 et 1000% tel que mesuré selon la méthode se trouvant sous le titre "Allongement à la rupture" dans la description.

7. Le timbre selon la revendication 1, où la température de transition vitreuse d'un élastomère formant le film élastomère est comprise entre -70°C et 20°C.

8. Le timbre selon la revendication 1, où le taux de transmission de la vapeur d'eau du timbre est de 1000 g/m²·24 h ou plus tel que mesuré selon la norme industrielle japonaise JIS Z 0238 dans les conditions de 40°C de température et de 90% d'humidité relative.

9. Le timbre selon la revendication 1, où au moins l'une de la couche de base et de la couche d'adhésif sensible à la pression contient un colorant.

10. Le timbre selon la revendication 1, où la transmittance des ultraviolets du timbre dans une plage de longueurs d'ondes comprise entre 280 et 400 nm est de 25% ou moins.

11. Le timbre selon la revendication 1, lequel a une structure stratifiée de "couche porteuse/couche de base/couche d'adhésif sensible à la pression/couche de séparation", dans lequel une couche porteuse est agencée sur la surface de la couche de base sur le côté opposé à la couche d'adhésif sensible à la pression et une couche de séparation est agencée sur la surface de la couche d'adhésif sensible à la pression sur le côté opposé à la couche de base.

12. Le timbre selon la revendication 1, où la surface de la couche de base sur le côté opposé à la couche d'adhésif sensible à la pression est en relief.
